# EUROPÄISCHE PATENTSCHRIFT

(11) **EP 1 832 220 B1**
(45) Veröffentlichungstag und Bekanntmachung des Hinweises auf die Patenterteilung: **29.08.2012**
(21) Anmeldenummer: 07003182.8
(22) Anmeldetag: 15.02.2007
(51) Int. Cl.: A61B 1/00, A61M 39/10, A61B 17/00

(54) **Medizintechnischer Kupplungsmechanismus zum Verbinden zweier medizinischer Instrumente**
Medical coupling mechanism for connecting two medical instruments
Mécanisme d'embrayage médical destiné au raccordement de deux instruments médicaux

(30) Priorität: 07.03.2006 DE 102006010316
(43) Veröffentlichungstag der Anmeldung: 12.09.2007
(73) Patentinhaber: Karl Storz GmbH & Co. KG, 78532 Tuttlingen (DE)
(72) Erfinder: Summerer, Sabine, 85551 Kirchheim (DE); Frey, Sebastian, 78054 Villingen-Schwenningen (DE); Sauer, Michael, 78532 Tuttlingen (DE)
(74) Vertreter: Hofmeister, Frank

(56) Entgegenhaltungen:
- EP-A- 0 990 831
- AT-B- 397 137
- DE-A1- 4 035 581
- DE-C1- 4 300 037
- US-A- 4 856 823
- US-A- 5 653 475
- US-A1- 2003 155 765
- US-A1- 2005 225 082

## Beschreibung

Die Erfindung betrifft einen medizintechnischen Kupplungsmechanismus zum Verbinden zweier medizinischer Instrumente, mit einem Kupplungsstecker und einer Kupplungsaufnahme zur Aufnahme des Kupplungssteckers, die über mindestens eine federbelastete Rastverbindung aneinander festlegbar sind, wobei die Rastverbindung aus mindestens zwei an einem der miteinander zu verbindenden Bauteile angeordneten und mit mindestens einem als Federring ausgebildeten Federelement in Wirkverbindung stehenden Rasthaken sowie mindestens einer am anderen Bauteil angeordneten Rastaufnahme zur Aufnahme der mindestens zwei Rasthaken besteht.

Ein gattungsgemäßer Kupplungsmechanismus ist aus der DE 43 00 037 C2 bekannt. Diese bekannte Steckverbindung besteht aus einem Stecker und einer Kupplung, wobei in die Kupplung ein Rasthaken aufweisendes Federelement so einsetzbar ist, dass die Rasthaken im verrasteten Zustand einen als Rastaufnahme dienenden Ring am Stecker hintergreifen.

US 2005/0225082 A1 offenbart einen medizintechnischen Kupplungsmechanismus entsprechend dem Oberbegriff der Ansprüche 1 und 14, wobei der Kupplungsmechanismus ein Rasthaken aufweisendes Federelement enthält, Rasthaken und Federelement sind dabei einstückig ausgebildet.

EP 0 990 831 A1 zeigt eine aus einem Stecker und einer Kupplung bestehende Steckverbindung, die als Rastaufnahme einen am Stecker angeformten umlaufenden Ring aufweist. Als Rasthaken dienen einzelne Rastelemente, die über einen außen an den Rasthaken anliegenden Federring an der Kupplung festlegbar sind, wobei der Federring in einer umlaufenden Vertiefung auf der Außenseite der Rastelemente gelagert ist.

Ein weiterer medizintechnischer Kupplungsmechanismus ist beilspielsweise aus der DE 44 25 705 C2 bekannt. Dieser bekannte Kupplungsmechanismus ist Bestandteil eines endoskopischen Instruments, wobei die Kupplungsaufnahme am proximalen Ende des Instruments ausgebildet ist und der Kupplungsstecker Bestandteil eines zweiten, mit diesem endoskopischen Instrument zu verbindenden Instruments ist. Dieser bekannte Kupplungsmechanismus besteht aus einer Bajonettkupplung sowie einem federbelasteten Rastelement, über das die beiden Bauteile gegeneinander fixierbar sind. Dieser bekannte Kupplungsmechanismus hat sich in der Praxis durchaus bewährt, jedoch ist der Aufbau mit einer Bajonettkupplung und einem Rastelement sehr aufwendig Darüber hinaus bedarf es einer gesonderten manuellen Verdrehung eines Drehrings, um die beiden Bauteile gegeneinander zu fixieren.

Davon ausgehend liegt der Erfindung die **Aufgabe** zugrunde, einen medizintechnischen Kupplungsmechanismus der eingangs genannten Art zu schaffen, der bei einfacher Handhabung eine sichere Verrastung der miteinander zu verbindenden Bauteile gewährleistet. Die Aufgabe wird durch die Merkmale der unabhängigen Ansprüche 1 und 14 gelöst.

Die **Lösung** dieser Aufgabenstellung ist erfingdungsgemäß dadurch gekennzeichnet, dass in den Rasthaken eine Nut zur Aufnahme des Federelements so ausgebildet ist, dass die Rasthaken auf dem Federelement aufsitzen.

Die Rasthaken sitzen somit auf dem Federring auf, wodurch eine direkte Wechseltwirkung zwischen der Federkraft des Federrings und der Beweglichkeit der Rasthaken gegeben ist.

Durch diese Ausgestaltung des medizintechnischen Kupplungsmechanismus ist es möglich, die beiden miteinander zu verbindenden Bauteile durch bloßes Zusammenstecken flüssigkeitsdicht miteinander zu verbinden. Einer zusätzlichen manuellen Tätigkeit oder eines Werkzeugeinsatzes bedarf es bei dem erfindungsgemäßen Mechanismus nicht.

Weiterhin wird mit der Erfindung vorgeschlagen, dass im Bereich oberhalb der Nut an den Rasthaken jeweils mindestens ein nach außen abstehender Steg ausgebildet ist, der bei in der Nut angeordnetem Federelement auf dem Federelement aufliegt. Durch diese auf dem Federelement aufliegenden Stege wird der Federring zur Lagefixierung immer nach unten gedrückt, so dass ein Verrutschen des Federrings nach oben beim zusammendrücken des Federrings nicht möglich ist.

Gemäß einer vorteilhaften Ausgestaltung der Erfindung wird vorgeschlagen, dass der Federring aus der Draufsicht eine elliptische Form aufweist. Die elliptische Form erlaubt eine besonders gute Verformbarkeit des Federrings durch seitliche Druckkräfte.

Um einen Federring bereitzustellen, der eine hohe Bruchsicherheit bei gleichzeitig hoher Federkraft aufweist, besteht der erfindungsgemäße Federring vorteilhafterweise aus Metall, vorzugsweise Federstahl.

Gemäß einer bevorzugten Ausführungsform des erfindungsgemäßen Kupplungsmechanismus wird vorgeschlagen, dass die Rasthaken an der Kupplungsaufnahme und die Rastaufnahme am Kupplungsstecker ausgebildet sind. Selbstverständlich ist es auch möglich den Kopplungsmechanismus so auszubilden, dass die Rasthaken am Kupplungsstecker und die Rastaufnahme an der Kupplungsaufnahme ausgebildet sind.

Zur Ausbildung der erfindungsgemäßen Rasthaken sind an den Rasthaken innen weisende Rastnasen ausgebildet, die im verrasteten Zustand in die als umlaufende Nut ausgebildete Rastaufnahme eingreifen.

Das Verrasten mit den Rasthaken kann erfindungsgemäß dadurch erleichtert werden, dass an der Oberseite der Rastnasen nach innen weisende Anlaufschrägen ausgebildet sind, wodurch die Rasthaken beim Anlaufen gegen das zu verrastende Bauteil automatisch nach außen gedrückt werden.

Um einerseits einen insbesondere axialen Toleranzausgleich bei der Ausgestaltung der Rasthaken und Rastaufnahmen zu schaffen und andererseits eine stets sichere Verrastung bei gleichzeitiger Flüssigkeitsdichtigkeit zu gewährleisten, wird mit der Erfindung vorgeschlagen, dass an der Unterseite der Rastnasen Fasen ausgebildet sind. Diese, vorteilhafterweise einen Winkel von 5° bis 15°, vorzugsweise 10°, gegenüber der Horizontalen aufspannenden Fasen, bewirken zusammen mit der Federkraft des Federrings, dass die Verrastung selbsthemmend immer in Richtung der Raststellung zusammengedrückt wird.

Zum Trennen der beiden über den erfindungsgemäßen Kupplungsmechanismus miteinander verbundenen Bauteile ist die Rastverbindung über einen mit dem mindestens einen Federelement in Wirkverbindung stehenden Auslösemechanismus wieder aufhebbar.

Gemäß einer ersten Ausführungsform der Erfindung wird vorgeschlagen, dass der Auslösemechanismus mindestens zwei an dem Federelement angeordnete Betätigungsknöpfe umfasst, über die das Federelement zusammendrückbar ist, wobei die Betätigungsknöpfe vorzugsweise versetzt zu den Rasthaken an dem Federelement angeordnet sind.

Gemäß einer alternativen zweiten Ausführungsform der Erfindung umfasst der Auslösemechanismus mindestens zwei Schieber, wobei jeder Schieber derart einstückig mit einem Rasthaken ausgebildet ist, dass ein Betätigungsteil des Schiebers um 180° versetzt zu dem jeweiligen Rasthaken am Schieber angeordnet ist.

Schließlich wird mit der Erfindung vorgeschlagen, dass am Kupplungsstecker und an der Kupplungsaufnahme miteinander korrespondierende Führungs- und/oder Positionierelemente ausgebildet sind, um eine lagegenaue und verdrehsichere Platzierung der Bauteile zueinander zu gewährleisten.

Weiterhin betrifft die Erfindung eine Kupplungsaufnahme zur Aufnahme eines Kupplungssteckers eines medizintechnischen Kupplungsmechanismus, insbesondere Instrumente für die endoskopische Chirurgie, wobei die Kupplungsaufnahme und der Kupplungsstecker über mindestens eine federbelastete Rastverbindung aneinander festlegbar sind und wobei die Rastverbindung aus mindestens zwei an der Kupplungsaufnahme angeordneten Rasthaken, die mit mindestens einem als Federring ausgebildeten Federelement in Wirkverbindung stehen, sowie mindestens einer am Kupplungsstecker angeordneten Rastaufnahme zur Aufnahme der mindestens zwei Rasthaken besteht, um so eine lagestabile und flüssigkeitsdichte Verrastung der Bauteile zu gewährleisten Da der Kupplungsstecker bei praktischen Ausführungsformen derartiger medizinischer Kupplungsmechanismen häufig an einem vielseitig verwendbaren Standard Instrument, beispielsweise einer Endoskop-Optik, ausgebildet ist, die über eine Kupplungsaufnahme mit verschiedenen anderen medizinischen Instrumenten koppelbar ist, zeichnet sich die erfindungsgemäße Kupplungsaufnahme dadurch aus, dass in den Rasthaken eine Nut zur Aufnahme des Federelements so ausgebildet ist, dass die Rasthaken auf dem Federelement aufsitzen.

Weitere Merkmale und Vorteile der Erfindung ergeben sich anhand der zugehörigen Zeichnung, in der zwei Ausführungsbeispiele eines erfindungsgemäßen medizintechnische Kupplungsmechanismus nur beispielhaft dargestellt sind, ohne die Erfindung auf diese Ausführungsbeispiele zu beschränken. In der Zeichnung zeigt:
- Fig. 1: eine perspektivische Ansicht einer ersten Ausführungsform eines erfindungsgemäßen medizintechnischen Kupplungsmechanismus, bestehend aus Kupplungsaufnahme und Kupplungsstecker, jedoch ohne Abdeckkappe für die Kupplungsaufnahme;
- Fig. 2: eine perspektivische Ansicht der Kupplungsaufnahme gemäß Fig. 1 ohne Kupplungsstecker;
- Fig. 3: eine perspektivische Ansicht gemäß Fig. 2, jedoch mit Abdeckkappe für die Kupplungsaufnahme;
- Fig. 4: eine vergrößerte perspektivische Ansicht des Details IV gemäß Fig. 1;
- Fig. 5a: eine geschnittene Seitenansicht des Kupplungsmechanismus gemäß Fig. 1 mit aufgesetzter Abdeckkappe vor dem Verrasten von Kupplungsaufnahme und Kupplungsstecker;
- Fig. 5b: eine ungeschnittene Draufsicht auf die Kupplungsaufnahme gemäß Fig. 5a, jedoch ohne Abdeckkappe und ohne Kupplungsstecker;
- Fig. 6a: eine Ansicht gemäß Fig. 5a, jedoch den Kupplungsmechanismus während des Einfügens des Kupplungssteckers darstellend;
- Fig. 6b: eine ungeschnittene Draufsicht auf die Kupplungsaufnahme gemäß Fig. 6a, jedoch ohne Abdeckkappe und ohne Kupplungsstecker;
- Fig. 7a: eine Ansicht gemäß Fig. 5a, jedoch den Kupplungsmechanismus im verrasteten Zustand von Kupplungsaufnahme und Kupplungsstecker darstellend;
- Fig. 7b: eine ungeschnittene Draufsicht auf die Kupplungsaufnahme gemäß Fig. 7a, jedoch ohne Abdeckkappe und ohne Kupplungsstecker;
- Fig. 8: eine vergrößerte Darstellung der Abbildung Fig. 7a;
- Fig. 9: eine perspektivische Ansicht einer zweiten Ausführungsform eines erfindungsgemäßen medizintechnischen Kupplungsmechanismus, bestehend aus Kupplungsaufnahme und Kupplungsstecker, jedoch ohne Abdeckkappe für die Kupplungsaufnahme;
- Fig. 10: eine perspektivische Ansicht der Kupplungsaufnahme gemäß Fig. 9 ohne Kupplungsstecker;
- Fig. 11: eine perspektivische Ansicht gemäß Fig. 10, jedoch mit Abdeckkappe für die Kupplungsaufnahme;
- Fig. 12: eine vergrößerte perspektivische Ansicht der Schieber gemäß Fig. 10;
- Fig. 13a: eine geschnittene Seitenansicht des Kupplungsmechanismus gemäß Fig. 9 vor dem Verrasten von Kupplungsaufnahme und Kupplungsstecker;
- Fig. 13b: eine ungeschnittene Draufsicht auf die Kupplungsaufnahme gemäß Fig. 13a, jedoch ohne Kupplungsstecker;
- Fig. 14a: eine Ansicht gemäß Fig. 13a, jedoch den Kupplungsmechanismus während des Einfügens des Kupplungssteckers darstellend;
- Fig. 14b: eine ungeschnittene Draufsicht auf die Kupplungsaufnahme gemäß Fig. 14a, jedoch ohne Kupplungsstecker;
- Fig. 15a: eine Ansicht gemäß Fig. 13a, jedoch den Kupplungsmechanismus im verrasten Zustand von Kupplungsaufnahme und Kupplungsstecker darstellend und
- Fig. 15b: eine ungeschnittene Draufsicht auf die Kupplungsaufnahme gemäß Fig. 15a, jedoch ohne Kupplungsstecker.

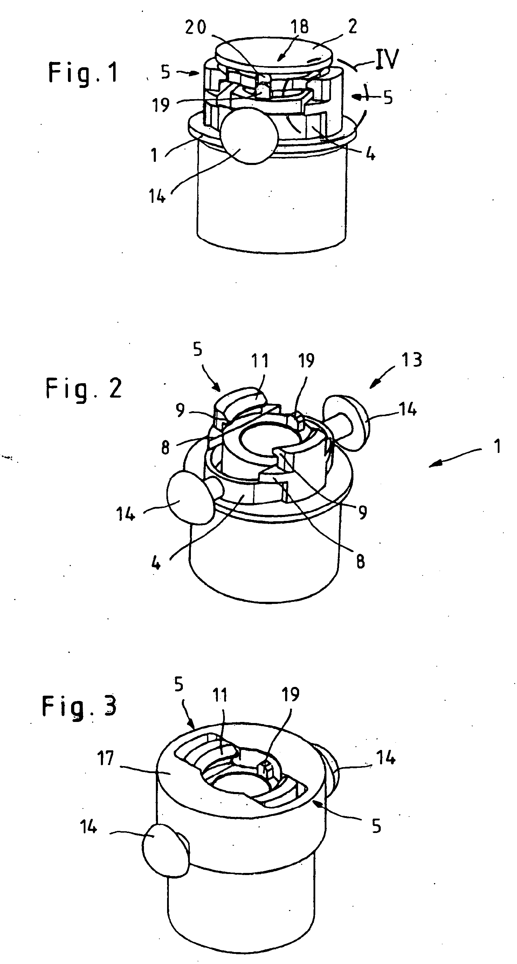

Die in den Abbildungen Fig. 1 bis Fig. 15b dargestellten medizintechnischen Kupplungsmechanismen bestehen im Wesentlichen aus einer Kupplungsaufnahme 1 und einem in der Kupplungsaufnahme 1 über eine Rastverbindung 3 verrastend festlegbaren Kupplungsstecker 2, wobei die Rastverbindung 3 aus mindestens zwei mit mindestens einem als Federring ausgebildeten Federelement 4 in Wirkverbindung stehenden Rasthaken 5 sowie mindestens einer Rastaufnahme 6 zur Aufnahme der mindestens zwei Rasthaken 5 besteht.

Bei den dargestellten Ausführungsbeispielen ist die Rastverbindung 3 jeweils so ausgebildet, dass die Rasthaken 5 an den Kupplungsaufnahmen 1 und die Rastaufnahmen 6 an den Kupplungssteckern 2 angeordnet sind. Selbstverständlich ist es auch möglich die Rastverbindung 3 so auszubilden, dass die Rasthaken 5 an den Kupplungssteckern 2 und die Rastaufnahmen 6 an den Kupplungsaufnahmen 1 angeordnet sind. Derartige Kupplungsmechanismen werden verwendet, um zwei medizinische Instrumente, beispielsweise ein endoskopisches Instrument und eine Endoskop-Optik, flüssigkeitsdicht miteinander zu verbinden.

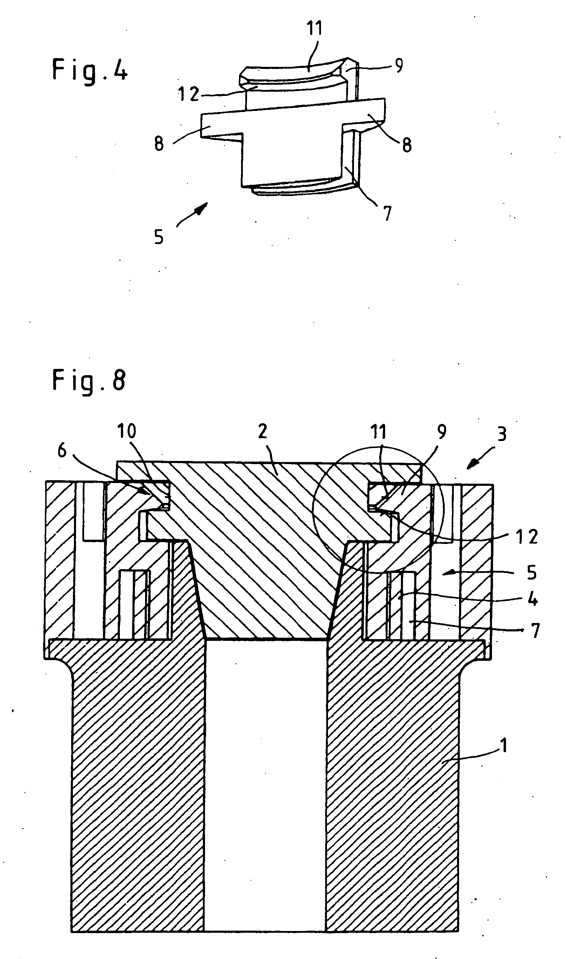

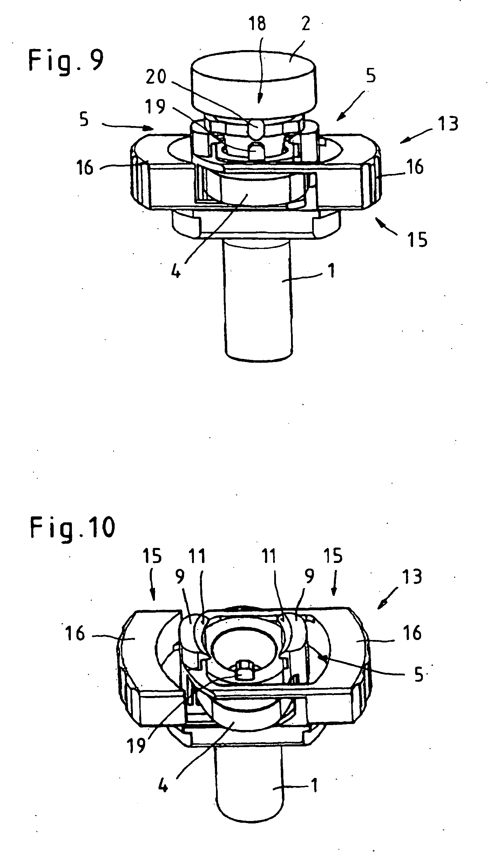

Wie aus den Abbildungen Fig. 4 in Zusammenschau mit den Abbildungen Fig. 1 und 2 sowie Fig. 12 in Zusammenschau mit den Abbildungen Fig. 9 und 10 ersichtlich, sind die Rasthaken 5 auf den Federring 4 aufgesetzt, wozu im Gehäuse der Rasthaken 5 eine Nut 7 zur Aufnahme des Federrings 4 ausgebildet ist. Um den in der Nut 7 gelagerten Federring 4 lagefest zu positionieren und ein Ausweichen nach oben zu verhindern, ist bei der in Fig. 4 dargestellten Ausführungsform der Rasthaken 5 im Bereich oberhalb der Nut 7 an den Rasthaken 5 beidseitig jeweils ein nach außen abstehender Steg 8 ausgebildet, der bei in der Nut 7 angeordnetem Federelement 4 auf dem Federelement 4 aufliegt. Weiterhin dienen die Stege 8 zur Lagerung der Rasthaken 5 in der Rastaufnahme 1.

Das eigentliche Verrasten der Rastverbindung 3 erfolgt über an den Rasthaken 5 ausgebildete, nach innen weisende Rastnasen 9, die im verrasteten Zustand in die als Nut 10 ausgebildete Rastaufnahme 6 eingreifen. Bei dem dargestellten Ausführungsbeispiel ist die Nut 10 als umlaufende Nut 10 ausgebildet. Zur Ausbildung der lagestabilen Verrastung ist es selbstverständlich ausreichend, nur abschnittweise Nuten 10 zur Aufnahme der jeweiligen Rastnasen 9 auszubilden.

Um das Einsetzen des Kupplungssteckers 2 in die Kupplungsaufnahme 1 zu erleichtern und das Verrasten der Rastnasen 9 in der Nut 10 zu vereinfachen, sind an der Oberseite der Rastnasen 9 nach innen weisende Anlaufschrägen 11 ausgebildet, die vorzugsweise einen Winkel von 45° aufweisen, wodurch die Rasthaken 5 beim Anlaufen gegen das zu verrastende Bauteil automatisch nach außen gedrückt werden.

Wie insbesondere aus vergrößerten Darstellung gemäß Fig. 8 ersichtlich, sind an der Oberseite der Rastnasen 9 nach innen weisende Fasen 12 ausgebildet, um einerseits einen Toleranzausgleich bei der Ausgestaltung der Rasthaken 5 und der Rastaufnahmen 6 zu schaffen und andererseits eine stets sichere und flüssigkeitsdichte Verrastung zu gewährleisten. Diese, vorteilhafterweise einen Winkel von 5° bis 15°, vorzugsweise 10°, gegenüber der Horizontalen aufspannenden Fasen 12, bewirken zusammen mit der Federkraft des Federrings 4, dass die Verrastung immer in Richtung der Raststellung zusammengedrückt wird.

Zum Trennen der beiden über den Kupplungsmechanismus miteinander verbundenen Bauteile 5 und 6 ist die Rastverbindung 3 über einen mit dem mindestens einen Federelement 4 in Wirkverbindung stehenden Auslösemechanismus 13 wieder aufhebbar.

Die in den Abbildungen Fig. 1 bis 8 dargestellte erste Ausführungsform des Kupplungsmechanismus und die in den Abbildungen Fig. 9 bis 15b dargestellte zweite Ausführungsform unterscheiden sich hinsichtlich der Ausgestaltung des Auslösemechanismus 13 voneinander.

Wie insbesondere aus Fig. 2 und Fig. 5a bis 7b ersichtlich, besteht der Auslösemechanismus 13 bei der ersten Ausführungsform aus zwei versetzt zu den Rasthaken 5 an dem Federelement 4 angeordneten Betätigungsknöpfen 14, über die das Federelement 4 zusammendrückbar ist.

Bei der dargestellten Ausgestaltungsform mit einem elliptischen Federring 4 sind die Rasthaken 5 und die Betätigungsknöpfe 14 des Auslösemechanismus 13 abwechselnd um jeweils 90° versetzt zueinander derart am Federring 4 angeordnet, dass die Rasthaken 5 einander gegenüberliegend an den näher beieinander liegenden Längsseiten des Federrings 4 angeordnet sind, wohingegen die Betätigungsknöpfe 14 einander gegenüberliegend an den weiter auseinander liegenden Längsseiten des Federrings 4 angeordnet sind.

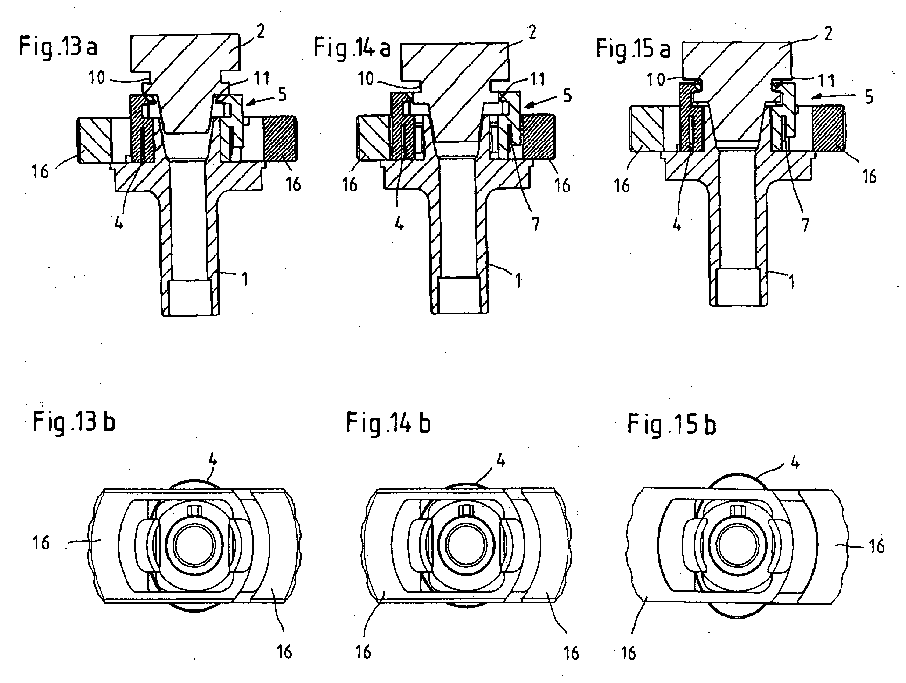

Wie insbesondere aus Fig. 10 und Fig. 13a bis 15b ersichtlich, besteht der Auslösemechanismus 13 bei der zweiten Ausführungsform aus mindestens zwei Schiebern 15, wobei jeder Schieber 15 derart einstückig mit einem Rasthaken 5 ausgebildet, dass ein Betätigungsteil 16 des Schiebers 15 um 180° versetzt zu dem jeweiligen Rasthaken 5 am Schieber 15 angeordnet ist. Wie aus Fig. 13a bis 15b ersichtlich, ist das Federelement 4 zur Aufhebung der Verrastung über die Schieber 15 auseinanderdrückbar.

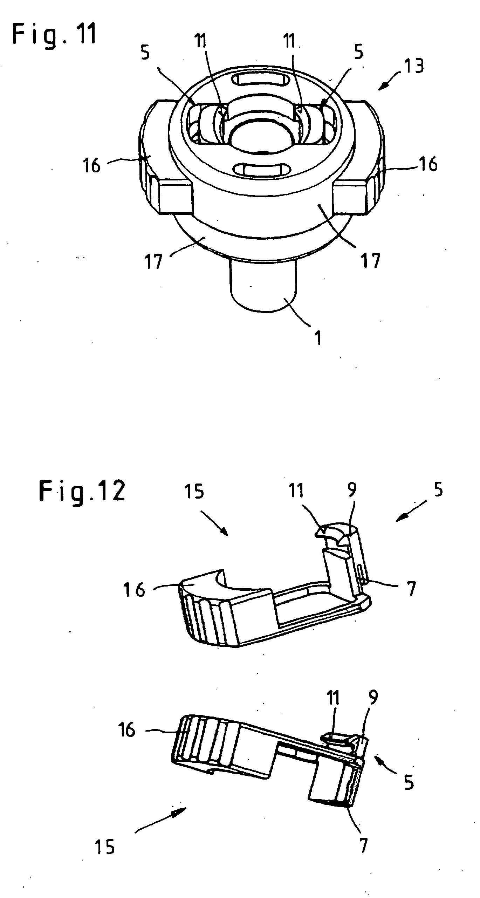

Wie weiterhin aus den Abbildungen Fig. 3 und Fig. 11 ersichtlich, sind die Kupplungsaufnahmen 1 über Abdeckkappen 17 so verschließbar, dass nur der Aufnahmebereich für den Kupplungsstecker 2 sowie der Bewegungsspielraum für die Rasthaken 5 frei bleibt. Während die Abdeckkappe 17 der in Fig. 3 dargestellten ersten Ausführungsform einteilig ausgebildet ist und beispielsweise mit der Kupplungsaufnahme 1 verschweißt oder verklebt wird, ist die Abdeckkappe 17 der in Fig. 11 dargestellten zweiten Ausführungsform zweiteilig ausgebildet. Die beiden Teile dieser Abdeckkappe lassen sich zum Abdecken der Kupplungsaufnahme 1 beispielsweise miteinander verschrauben. Ebenso ist es selbstverständlich auch möglich, die Abdeckkappe 17 der ersten Ausführungsform zweiteilig auszugestalten.

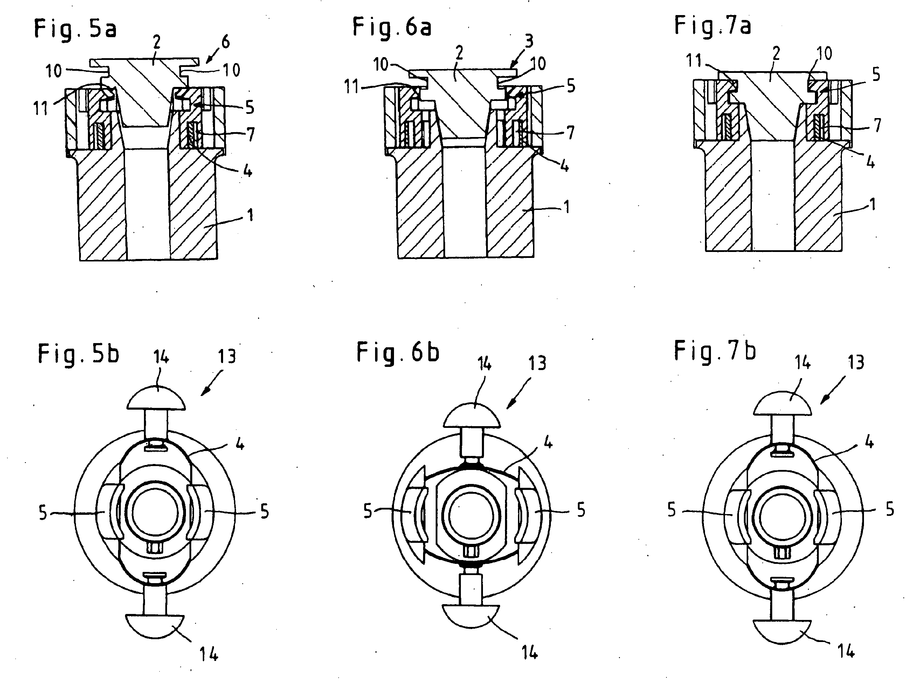

Das Betätigen des Kupplungsmechanismus wird nachfolgend anhand der Abbildungen Fig. 5a bis 7b und 13a bis 15b erläutert:

Um eine lagegenaue und verdrehsichere Platzierung der Bauteile Kupplungsaufnahme 1 und Kupplungsstecker 2 zueinander zu gewährleisten, sind, wie aus Fig. 1 und 9 ersichtlich, am Kupplungsstecker 2 und an der Kupplungsaufnahme 1 miteinander korrespondierende Führungs- und/oder Positionierelemente 18 ausgebildet, die bei der dargestellten Ausführungsform als Zentrierzapfen 19 und Aufnahmenut 20 ausgebildet sind.

Die Abbildungen Fig. 5a und 13a zeigen die Ausgangsstellung vor der beiderseitigen Verrastung von Kupplungsaufnahme 1 und Kupplungsstecker 2. In dieser Stellung liegt der Kupplungsstecker 1 oberhalb der Anlaufschrägen 11 auf den Rastnasen 9 auf, wobei die Anlaufschrägen 11 mit einem Winkel von etwa 45° nach innen weisend ausgebildet sind.

Sobald nunmehr eine die beiden Bauteile 1 und 2 aufeinander zu bewegende, im Wesentlichen axial wirkende Druckkraft auf den Kupplungsstecker 2 und/oder die Kupplungsaufnahme 1 ausgeübt wird, bewirken die Anlaufschrägen 11 der Rastnasen 9 der Rasthaken 5, dass die Rasthaken 5 gegen die Kraft des Federrings 4 so weit nach außen gedrückt werden, bis der Kupplungsstecker 2 die in Fig. 6a und 14a dargestellte Zwischenstellung eingenommen hat.

Durch weitere Druckausübung wird der Kupplungsstecker 2 in die in Fig. 7a und 15a dargestellte Raststellung überführt, in der die Rastnasen 9 der Rasthaken 5 in die Nut 10 der Rastaufnahme 6 eingreifen. Wie insbesondere aus Fig. 8 ersichtlich, bewirken die an der Unterseite der Rastnasen 9 ausgebildeten Fasen 12 in dieser Raststellung zusammen mit der Federspannung des Federrings 4, dass die Rastverbindung 3 selbsthemmend und flüssigkeitsdicht fest in die verrastete Stellung gedrückt wird.

Die Verformung des Federrings 4 während des Verrastungsvorgangs lässt sich gut anhand der Draufsichten gemäß Fig. 5b bis 7b und 13b bis 15b erkennen.

Zum Lösen der Rastverbindung wird bei der in Fig. 1 bis 8 dargestellten ersten Ausführungsform der Federring 4 über die Betätigungsknöpfe 14 zusammengedrückt, bis die Rastnasen 9 der Rasthaken 5 außer Eingriff mit der Nut 10 der Rastaufnahmen 6 treten.

Bei der in Fig. 9 bis 15b dargestellten zweiten Ausführungsform wird der Federring 4 durch Druckausübung auf die Betätigungsteile 16 der Schieber 15 auseinandergedrückt, bis die einteilig mit den Schiebern 15 ausgebildeten Rasthaken 5 so weit nach außen verlagert wurden, dass die Rastnasen 9 außer Eingriff mit der Nut 10 treten.

Solchermaßen ausgestaltete medizintechnische Kupplungsmechanismen zeichnen sich dadurch aus, dass diese durch bloßes Zusammendrücken der miteinander zu verbindenden Bauteile 1 und 2 eine flüssigkeitsdichte Verbindung bewirken.

### Bezugszeichenliste

- 1: Kupplungsaufnahme
- 2: Kupplungsstecker
- 3: Rastverbindung
- 4: Federelement/Federring
- 5: Rasthaken
- 6: Rastaufnahme
- 7: Nut (Rasthaken)
- 8: Steg
- 9: Rastnase
- 10: Nut (Rastaufnahme)
- 11: Anlaufschräge
- 12: Fase
- 13: Auslösemechanismus
- 14: Betätigungsknopf
- 15: Schieber
- 16: Betätigungsteil
- 17: Abdeckkappe
- 18: Führungs-/Positionierelement
- 19: Zentrierzapfen
- 20: Aufnahmenut

## Patentansprüche

1. Medizintechnischer Kupplungsmechanismus zum Verbinden zweier medizinischer Instrumente, mit einem Kupplungsstecker (2) und einer Kupplungsaufnahme (1) zur Aufnahme des Kupplungssteckers (2), die über mindestens eine federbelastete Rastverbindung (3) aneinander festlegbar sind, wobei die Rastverbindung (3) aus mindestens zwei an einem der miteinander zu verbindenden Bauteile (1 oder 2) angeordneten und mit mindestens einem als Federring ausgebildeten Federelement (4) in Wirkverbindung stehenden Rasthaken (5) sowie mindestens einer am anderen Bauteil (2 oder 1) angeordneten Rastaufnahme (6) zur Aufnahme der mindestens zwei Rasthaken (5) besteht,
**dadurch gekennzeichnet,**
**dass** in den Rasthaken (5) eine Nut (7) zur Aufnahme des Federelements (4) so ausgebildet ist, dass die Rasthaken (5) auf dem Federelement (4) aufsitzen.

2. Medizintechnischer Kupplungsmechanismus nach Anspruch 1, **dadurch gekennzeichnet, dass** im Bereich oberhalb der Nut (7) an den Rasthaken (5) jeweils mindestens ein nach außen abstehender Steg (8) ausgebildet ist, der bei in der Nut (7) angeordnetem Federelement (4) auf dem Federelement (4) aufliegt.

3. Medizintechnischer Kupplungsmechanismus nach Anspruch 1 oder 2, **dadurch gekennzeichnet, dass** der Federring (4) aus der Draufsicht eine elliptische Form aufweist.

4. Medizintechnischer Kupplungsmechanismus nach Anspruch 3, **dadurch gekennzeichnet, dass** das mindestens eine Federelement (4) aus Metall, vorzugsweise Federstahl, besteht.

5. Medizintechnischer Kupplungsmechanismus nach einem der Ansprüche 1 bis 4, **dadurch gekennzeichnet, dass** die Rasthaken (5) an der Kupplungsaufnahme (1) und die Rastaufnahme (6) am Kupplungsstecker (2) ausgebildet sind.

6. Medizintechnischer Kupplungsmechanismus nach einem der Ansprüche 1 bis 5, **dadurch gekennzeichnet, dass** an den Rasthaken (5) nach innen weisende Rastnasen (9) ausgebildet sind, die im verrasteten Zustand in die als Nut (10), insbesondere als umlaufende Nut, ausgebildete Rastaufnahme (6) eingreifen.

7. Medizintechnischer Kupplungsmechanismus nach Anspruch 6, **dadurch gekennzeichnet, dass** an der Oberseite der Rastnasen (9) nach innen weisende Anlaufschrägen (11) ausgebildet sind.

8. Medizintechnischer Kupplungsmechanismus nach Anspruch 6 oder 7, **dadurch gekennzeichnet, dass** an der Unterseite der Rastnasen (9) Fasen (12) ausgebildet sind.

9. Medizintechnischer Kupplungsmechanismus nach Anspruch 8, **dadurch gekennzeichnet, dass** die Fasen (12) einen Winkel von 5° bis 15° gegenüber der Horizontalen aufspannt.

10. Medizintechnischer Kupplungsmechanismus nach einem der Ansprüche 1 bis 9, **dadurch gekennzeichnet, dass** die Rastverbindung (3) über einen mit dem mindestens einen Federelement (4) in Wirkverbindung stehenden Auslösemechanismus (13) wieder aufhebbar ist.

11. Medizintechnischer Kupplungsmechanismus nach Anspruch 10, **dadurch gekennzeichnet, dass** der Auslösemechanismus (13) mindestens zwei an dem Federelement (4) angeordnete Betätigungsknöpfe (14) umfasst, über die das Federelement (4) zusammendrückbar ist.

12. Medizintechnischer Kupplungsmechanismus nach Anspruch 10, **dadurch gekennzeichnet, dass** der Auslösemechanismus (13) mindestens zwei Schieber (15) umfasst, wobei jeder Schieber (15) derart einstückig mit einem Rasthaken (5) ausgebildet, dass ein Betätigungsteil (16) des Schlebers (15) um 180° versetzt zu dem jeweiligen Rasthaken (5) am Schleber (15) angeordnet ist.

13. Medizintechnischer Kupplungsmechanismus nach einem der Ansprüche 1 bis 12, **dadurch gekennzeichnet, dass** am Kupplungsstecker (2) und an der Kupplungsaufnahme (1) miteinander korrespondierende Führungs- und/oder Positionierelemente (18) ausgebildet sind.

14. Kupplungsaufnahme zur Aufnahme eines Kupplungssteckers (2) eines medizintechnischen Kupplungsmechanismus, wobei die Kupplungsaufnahme (1) und der Kupplungsstecker (2) über mindestens eine federbelastete Rastverbindung (3) aneinander festlegbar sind, wobei die Rastverbindung (3) aus mindestens zwei an der Kupplungsaufnahme (1) angeordneten Rasthaken (5), die mit mindestens einem als Federring ausgebildeten Federelement (4) in Wirkverbindung stehen, sowie mindestens einer am Kupplungsstecker (2) angeordneten Rastaufnahme (6) zur Aufnahme der mindestens zwei Rasthaken (5) besteht,
**dadurch gekennzeichnet,**
**dass** in den Rasthaken (5) eine Nut (7) zur Aufnahme des Federelements (4) so ausgebildet ist, dass die Rasthaken (5) auf dem Federelement (4) aufsitzen.

## Claims

1. A medical coupling mechanism for connecting two medical instruments having a coupling plug (2) and a coupling receptacle (1) for receiving the coupling plug (2) which can be fixed to one another by means of at least one spring-loaded latch connection (3), wherein the latch connection (3) consists of at least two latching hooks (5), which are arranged on one of the components (1 or 2) to be connected to one another and are actively connected to at least one spring element (4) in the form of a spring ring, and at least one latching receptacle (6), which is arranged on the other component (2 or 1) for receiving the at least two latching hooks (5),
**characterized in that**
a slot (7) for receiving the spring element (4) is formed in the latching hooks (5) so that the latching hooks (5) rest on the spring element (4).

2. The medical coupling mechanism as claimed in claim 1, **characterized in that** in each case at least one outwardly projecting land (8), which rests on the spring element (4) when the spring element (4) is arranged in the slot (7), is formed on the latching hooks (5) in the region above the slot (7).

3. The medical coupling mechanism as claimed in claim 1 or 2, **characterized in that**, in plan view, the spring ring (4) has an elliptical shape.

4. The medical coupling mechanism as claimed in claim 3, **characterized in that** the at least one spring element (4) is made of metal, preferably spring steel.

5. The medical coupling mechanism as claimed in one of claims 1 to 4, **characterized in that** the latching hooks (5) are formed on the coupling receptacle (1) and the latching receptacle (6) on the coupling plug (2).

6. The medical coupling mechanism as claimed in one of claims 1 to 5, **characterized in that** inwardly facing latching lugs (9), which in the latched state engage in the latching receptacle (6) formed as a slot (10) and in particular a circumferential slot, are formed on the latching hooks (5).

7. The medical coupling mechanism as claimed in claim 6, **characterized in that** inwardly facing leading chamfers (11) are formed on the top of the latching lugs (9).

8. The medical coupling mechanism as claimed in claim 6 or 7, **characterized in that** chamfers (12) are formed on the bottom of the latching lugs (9).

9. The medical coupling mechanism as claimed in claim 8, **characterized in that** the chamfers (12) span an angle of 5° to 15° with respect to the horizontal.

10. The medical coupling mechanism as claimed in one of claims 1 to 9, **characterized in that** the latch connection (3) can be released by means of a release mechanism (13) which is actively connected to the at least one spring element (4).

11. The medical coupling mechanism as claimed in claim 10, **characterized in that** the release mechanism (13) comprises at least two actuating knobs (14) arranged on the spring element (4), by means of which the spring element (4) can be pressed together.

12. The medical coupling mechanism as claimed in claim 10, **characterized in that** the release mechanism (13) comprises at least two sliders (15), wherein each slider (15) is formed in one piece with a latching hook (5) in such a way that an actuating part (16) of the slider (15) is arranged on the slider (15) offset by 180° relative to the respective latching hook (5).

13. The medical coupling mechanism as claimed in one of claims 1 to 12, **characterized in that** mutually corresponding guide and/or positioning elements (18) are formed on the coupling plug (2) and on the coupling receptacle (1).

14. A receptacle for receiving a coupling plug (2) of a medical coupling mechanism, wherein the coupling receptacle (1) and the coupling plug (2) can be fixed to one another by means of at least one spring-loaded latch connection (3), wherein the latch connection (3) consists of at least two latching hooks (5), which are arranged on the coupling receptacle (1) and are actively connected to at least one spring element (4) in the form of a spring ring, and at least one latching receptacle (6), which is arranged on the coupling plug (2) for receiving the at least two latching hooks (5),
**characterized in that**
a slot (7) for receiving the spring element (4) is formed in the latching hooks (5) so that the latching hooks (5) rest on the spring element (4).

## Revendications

1. Mécanisme de couplage du domaine technique médical, destiné à relier deux instruments médicaux, comprenant un embout mâle de couplage (2) et un logement récepteur de couplage (1) destiné à recevoir l'embout mâle de couplage (2), qui peuvent être fixés l'un à l'autre par l'intermédiaire d'au moins une liaison par encliquetage (3) chargée par ressort, la liaison par encliquetage (3) étant constituée d'au moins deux crochets d'encliquetage (5), qui sont agencés sur l'une des deux pièces (1 ou 2) à relier et sont en liaison interactive avec au moins un élément de ressort (4) réalisé en tant qu'anneau de ressort, ainsi que d'au moins un logement d'encliquetage (6) agencé sur l'autre pièce (2 ou 1) et destiné à recevoir lesdits au moins deux crochets d'encliquetage (5),
**caractérisé en ce que** dans les crochets d'encliquetage (5) est réalisée une rainure (7) destinée à recevoir l'élément de ressort (4) de manière telle que les crochets d'encliquetage (5) reposent sur l'élément de ressort (4).

2. Mécanisme de couplage du domaine technique médical selon la revendication 1, **caractérisé en ce que** dans la zone au-dessus de la rainure (7), sur les crochets d'encliquetage (5) est respectivement formé au moins un appendice (8) qui fait saillie vers l'extérieur et repose sur l'élément de ressort (4) lorsque l'élément de ressort (4) est agencé dans la rainure (7).

3. Mécanisme de couplage du domaine technique médical selon la revendication 1 ou la revendication 2, **caractérisé en ce que** l'anneau de ressort (4) présente une forme elliptique en vue de dessus.

4. Mécanisme de couplage du domaine technique médical selon la revendication 3, **caractérisé en ce que** ledit au moins un élément de ressort (4) est réalisé en métal, de préférence en acier à ressort.

5. Mécanisme de couplage du domaine technique médical selon l'une des revendications 1 à 4, **caractérisé en ce que** les crochets d'encliquetage (5) sont formés sur le logement récepteur de couplage (1), et le logement d'encliquetage (6) est formé sur l'embout mâle de couplage (2).

6. Mécanisme de couplage du domaine technique médical selon l'une des revendications 1 à 5, **caractérisé en ce que** sur les crochets d'encliquetage (5) sont formés des talons d'encliquetage (9) qui, dans l'état encliqueté, s'engagent dans le logement d'encliquetage (6) réalisé sous forme de rainure (10), notamment sous forme de rainure périphérique.

7. Mécanisme de couplage du domaine technique médical selon la revendication 6, **caractérisé en ce que** sur le côté supérieur des talons d'encliquetage (9) sont formées des rampes inclinées (11) dirigées vers l'intérieur.

8. Mécanisme de couplage du domaine technique médical selon la revendication 6 ou la revendication 7, **caractérisé en ce que** sur le côté inférieur des talons d'encliquetage (9) sont formés des chanfreins (12).

9. Mécanisme de couplage du domaine technique médical selon la revendication 8, **caractérisé en ce que** les chanfreins (12) forment un angle de 5° à 15° par rapport à l'horizontale.

10. Mécanisme de couplage du domaine technique médical selon l'une des revendications 1 à 9, **caractérisé en ce que** la liaison par encliquetage (3) peut à nouveau être interrompue par l'intermédiaire d'un mécanisme de déclenchement (13) en liaison interactive avec ledit au moins un élément de ressort (4).

11. Mécanisme de couplage du domaine technique médical selon la revendication 10, **caractérisé en ce que** le mécanisme de déclenchement (13) englobe au moins deux boutons d'actionnement (14) agencés sur l'élément de ressort (4), par l'intermédiaire desquels l'élément de ressort (4) peut être comprimé.

12. Mécanisme de couplage du domaine technique médical selon la revendication 10, **caractérisé en ce que** le mécanisme de déclenchement (13) englobe au moins deux coulisseaux (15), chaque coulisseau (15) étant réalisé d'un seul tenant avec un crochet d'encliquetage (5) de façon telle qu'une pièce d'actionnement (16) du coulisseau (15) soit agencée sur le coulisseau (15) de manière décalée de 180° par rapport au crochet d'encliquetage (5) respectivement correspondant.

13. Mécanisme de couplage du domaine technique médical selon l'une des revendications 1 à 12, **caractérisé en ce que** sur l'embout mâle de couplage (2) et sur le logement récepteur de couplage (1) sont formés des éléments de guidage et/ou de positionnement (18) mutuellement correspondants.

14. Logement récepteur de couplage destiné à recevoir un embout mâle de couplage (2) d'un mécanisme de couplage du domaine technique médical, le logement récepteur de couplage (1) et l'embout mâle de couplage (2) pouvant être fixés l'un à l'autre par l'intermédiaire d'au moins une liaison par encliquetage (3) chargée par ressort, et la liaison par encliquetage (3) étant constituée d'au moins deux crochets d'encliquetage (5), qui sont agencés sur le logement récepteur de couplage (1) et sont en liaison interactive avec au moins un élément de ressort (4) réalisé en tant qu'anneau de ressort, ainsi que d'au moins un logement d'encliquetage (6) agencé sur l'embout mâle de couplage (2) et destiné à recevoir lesdits au moins deux crochets d'encliquetage (5),
**caractérisé en ce que** dans les crochets d'encliquetage (5) est réalisée une rainure (7) destinée à recevoir l'élément de ressort (4) de manière telle que les crochets d'encliquetage (5) reposent sur l'élément de ressort (4).
